# FASCICULE DE BREVET EUROPEEN

(11) **EP 0 603 231 B1**
(45) Date de publication et mention de la délivrance du brevet: **03.01.1996**
(21) Numéro de dépôt: 92918576.7
(22) Date de dépôt: 02.09.1992
(51) Int. Cl.: C07C 2/72, B01J 23/04, B01J 31/12

(54) **PROCEDE POUR LA SYNTHESE D'HYDROCARBURES AROMATIQUES ALKYLES**
VERFAHREN ZUR SYNTHESE VON ALKYLIERTEN AROMATISCHEN KOHLENWASSERSTOFFEN
METHOD FOR SYNTHESIZING ALKYLATED AROMATIC HYDROCARBONS

(30) Priorité: 12.09.1991 BE 9100850
(43) Date de publication de la demande: 29.06.1994
(73) Titulaire: SOLVAY INTEROX, B-1050 Bruxelles (BE)
(72) Inventeur: GANHY, Jean-Pierre, B-1170 Bruxelles (BE)
(74) Mandataire: Decamps, Alain René François
(86) Numéro de dépôt international: EP9202026
(87) Numéro de publication internationale: WO9305001

(56) Documents cités:
- WO-A-90/14323
- FR-A- 2 647 438
- GB-A- 1 259 535

## Description

La présente invention concerne un procédé pour la fabrication d'hydrocarbures aromatiques alkylés. Elle concerne plus particulièrement la fabrication d'hydrocarbures aromatiques alkylés à chaîne alkyle comprenant au moins quatre atomes de carbone par condensation d'un hydrocarbure aromatique substitué par un groupement alkyle à chaîne courte comportant 1 à 3 atomes de carbone avec une oléfine en présence d'un catalyseur.

Le brevet GB-A-1259535 (BP CHEMICALS) divulgue un procédé de production d'un hydrocarbure aromatique alkylé par alkylation d'une chaîne latérale qui comprend l'alkylation d'un hydrocarbure aromatique ayant une chaîne latérale contenant un atome d'hydrogène sur un atome de carbone en position alpha par rapport au noyau benzénique au moyen d'une oléfine en présence d'un catalyseur comprenant un métal alcalin ou un hydrure de métal alcalin déposé sur de l'alumine (revendication 1). Dans ce procédé, le catalyseur est préparé hors du milieu réactionnel avant la réaction d'alkylation, par traitement thermique (exemple 1, page 2, lignes 15 à 20).

La demande de brevet FR-A-2647438 et la demande de brevet internationale WO-A-9014323 (INTEROX) divulguent un procédé de synthèse d'hydrocarbures aromatiques alkylés consistant à faire réagir un hydrocarbure aromatique substitué par un groupement alkyle à chaîne courte avec une oléfine en présence d'un catalyseur préparé dans le milieu réactionnel par mélange d'alumine anhydre avec un métal alcalin ou un hydrure de métal alcalin. De manière préférée, le rapport pondéral métal alcalin/alumine ou hydrure de métal alcalin/alumine est compris entre 0,02 et 0,5.

Il a été trouvé que, de manière surprenante, il était possible d'augmenter fortement la productivité de la réaction si l'on réglait le rapport pondéral métal alcalin/alumine ou hydrure de métal alcalin/alumine pour qu'il se situe dans une plage de valeurs plus élevées.

A cet effet, l'invention concerne un procédé de synthèse d'hydrocarbures aromatiques alkylés à chaîne alkyle saturée comprenant au moins quatre atomes de carbone selon lequel on fait réagir un hydrocarbure aromatique substitué par un groupement alkyle à chaîne courte saturée comportant un à trois atomes de carbone avec une oléfine en présence d'un catalyseur qui comprend au moins un métal alcalin ou un hydrure de métal alcalin imprégné sur un support d'alumine, le catalyseur étant préparé dans le milieu réactionnel en présence de l'hydrocarbure aromatique à courte chaîne alkyle par mélange de l'alumine anhydre avec le métal alcalin ou avec l'hydrure de métal alcalin; selon l'invention, le rapport pondéral du métal alcalin ou de l'hydrure de métal alcalin au support d'alumine est compris entre 0,6 et 1,8.

L'invention vise à préparer des hydrocarbures aromatiques alkylés par une chaîne alkyle saturée linéaire ou ramifiée comprenant plus de quatre et, de préférence, pas plus de huit atomes de carbone. Elle s'adresse en particulier à la préparation d'hydrocarbures benzéniques alkylés par une chaîne alkyle saturée et ramifiée comprenant cinq atomes de carbone. Elle est tout particulièrement adaptée à la préparation d'amylbenzènes comme, par exemple, le tert-amylbenzène.

Par hydrocarbure aromatique alkylé à chaîne courte utilisé comme matière première selon l'invention, on entend désigner tout hydrocarbure aromatique comportant un ou plusieurs cycles aromatiques à cinq ou six atomes de carbone et au moins une chaîne alkyle latérale saturée comprenant un à trois atomes de carbone. Les hydrocarbures benzéniques à un seul noyau aromatique sont préférés et tout particulièrement le toluène, les diméthyl et triméthylbenzène, l'éthylbenzène, les éthyltoluènes, le n-propylbenzène et le cumène.

L'oléfine utilisée comme agent d'alkylation doit comporter une chaîne carbonée linéaire comprenant au moins deux et pas plus de cinq atomes de carbone, deux au moins d'entre eux étant liés par une liaison insaturée de type oléfinique. Les monooléfines ne présentant qu'une seule insaturation sont préférées. A titre d'exemples, les oléfines suivantes peuvent être utilisées dans le procédé selon l'invention : l'éthylène, le propylène, les n-butènes, l'isobutène, le butadiène, les n-pentènes et les méthylbutènes.

Le catalyseur mis en oeuvre dans le procédé selon l'invention doit comporter au moins un métal alcalin ou un hydrure de métal alcalin en plus du support d'alumine. Tout métal alcalin peut convenir. Le sodium ou l'hydrure de sodium sont cependant généralement préférés en raison de leur bonne activité et de leur disponibilité élevée. Le sodium peut être le seul métal alcalin présent avec le support d'alumine; un tel catalyseur a donné d'excellents résultats.

Une variante du catalyseur conforme au procédé selon l'invention consiste à mettre en oeuvre un support d'alumine et un mélange de métaux alcalins ou d'hydrures de métaux alcalins dans des proportions sensiblement égales ou, dans une autre variante, telles qu'un seul des métaux alcalins ou des hydrures de métaux alcalins n'excède pas le double de la proportion de tous les autres métaux alcalins ou hydrures de métaux alcalins réunis.

Une autre variante particulièrement intéressante du catalyseur conforme au procédé selon l'invention consiste à mélanger àun métal alcalin principal ou à un hydrure de métal alcalin de petites quantités n'excédant pas quelques pour cent d'un ou de deux autres métaux alcalins ou hydrures de métaux alcalins à titre de promoteur et de les additionner au support d'alumine. Le mélange de sodium ou d'hydrure de sodium avec plus de 0,1 % de rubidium et/ou de césium ou d'hydrure de rubidium et/ou de césium est tout particulièrement digne d'intérêt. De même il est avantageux que le mélange de sodium ou d'hydrure de sodium avec le rubidium et/ou le césium ou l'hydrure de rubidium et/ou l'hydrure de césium contienne moins de 5 % de rubidium et/ou de césium ou moins de 5 % d'hydrure de rubidium et/ou d'hydrure de césium.

Il est aussi possible de combiner dans un même catalyseur un mélange de métal alcalin avec un hydrure de métal alcalin.

De manière préférée, les quantités de métal alcalin ou d'hydrure de métal alcalin mises en oeuvre sont telles que le catalyseur présente un rapport pondéral métal alcalin/alumine ou hydrure de métal alcalin/alumine supérieur à 0,7. Il est aussi avantageux que ce rapport pondéral soit inférieur à 1,5.

Selon l'invention, le support d'alumine peut être constitué d'alumine de différentes variétés cristallines pures ou en mélange. Les variétés d'alumine α, β et γ conviennent bien. De bons résultats ont été obtenus avec un support uniquement constitué d'alumine γ.

Les alumines utilisées comme support du catalyseur dans le procédé selon l'invention sont des substances poreuses. Elles présentent généralement un diamètre moyen de pores supérieur à 0,1 nm. De même, ce diamètre moyen de pores est souvent inférieur à 500 nm. Des résultats intéressants ont été obtenus avec des alumines dont le diamètre moyen des pores était plus grand que 2 nm. De bons résultats ont aussi été obtenus avec des alumines dont le diamètre moyen des pores était inférieur à 100 nm. Les meilleurs résultats ont été obtenus en mettant en oeuvre des alumines de 20 et 30 nm de diamètre moyen de pores.

Pour que le catalyseur obtenu par mélange d'un métal alcalin ou d'un hydrure de métal alcalin avec ces alumines soit efficace, il faut généralement que la surface spécifique de ces alumines soit supérieure à 10 m/g. Il convient aussi que la surface spécifique de ces alumines soit inférieure à 360 m/g. En pratique, on préfère les alumines dont la surface spécifique est supérieure à 50 m/g. De même les alumines dont la surface spécifique est inférieure à 200 m/g sont préférées.

Généralement, les alumines qui conviennent pour accompagner les métaux alcalins dans les catalyseurs conformes au procédé selon l'invention présentent un volume de pores supérieur à 25 ml/100 g et, de préférence, supérieur à 45 ml/100 g. Le plus souvent, le volume de pores de ces alumines est inférieur à 75 ml/100 g et, de préférence, inférieur à 65 ml/100 g.

Les alumines mises en oeuvre doivent être exemptes de traces d'eau libre. Au besoin, il peut être utile de leur faire subir préalablement à leur mise en oeuvre comme support du métal alcalin une calcination à une température supérieure à 200 °C dans le but d'éliminer toute trace d'humidité résiduelle. La température de calcination de ces alumines ne dépasse le plus souvent pas 600 °C.

Les quantités de catalyseur optimales ne sont pas critiques. Pour assurer une bonne efficacité du catalyseur, il convient cependant que le rapport pondéral du métal alcalin à l'hydrocarbure aromatique à courte chaîne soit supérieur à 0,0015. De même, il est avantageux que ce rapport pondéral reste inférieur à 0,03.

Une variante intéressante du procédé selon l'invention consiste à ajouter un promoteur tel que l'hydroxyde de potassium lors de la préparation du catalyseur dans le milieu réactionnel. L'hydroxyde de potassium est généralement mis en oeuvre en quantité telle que le rapport pondéral hydroxyde de K / métal alcalin ou hydrure de métal alcalin est égal ou supérieur à 0,3 et, de préférence, égal ou supérieur à 0,5. Ce rapport pondéral est aussi souvent inférieur à 2 et, de préférence, à 1,5.

La température et la pression auxquelles on effectue la réaction d'alkylation conforme au procédé selon l'invention doivent être adaptées à la nature de l'oléfine et de l'hydrocarbure aromatique de départ. On a cependant constaté que des pressions supérieures à 0,5 MPa et des températures supérieures à 110 °C conviennent bien. De même, on a constaté que les pressions adéquates sont généralement inférieures à 10 MPa et que les températures se situent souvent en-dessous de 250 °C. Des pressions supérieures à 1 MPa et des températures supérieures à 170 °C ont donné les meilleurs résultats. Ceux-ci sont aussi obtenus avec des pressions inférieures à 5 MPa et des températures plus basses que 200 °C et, de préférence plus basses ou égales à 198°C. Lorsque l'on effectue la réaction en phase vapeur, il est avantageux de fixer les pressions respectives de l'oléfine et de l'hydrocarbure aromatique de départ de manière telle que le rapport molaire oléfine/hydrocarbure aromatique soit supérieur à 0,7. De même, dans ce cas, il est aussi intéressant que ce rapport molaire soit inférieur à 1,3. De préférence, on choisira un rapport molaire oléfine/hydrocarbure aromatique voisin de 1.

Afin d'assurer un bon contact entre les réactifs, il est généralement nécessaire d'effectuer la réaction sous une agitation vigoureuse, en particulier lorsque l'hydrocarbure de départ est liquide et l'oléfine est gazeuse dans les conditions de la réaction. Tous les types d'agitateurs connus conviennent généralement bien. Le choix d'un agitateur particulier s'effectue en fonction du type d'appareillage utilisé pour le réacteur.

La réaction conforme au procédé selon l'invention peut indifféremment s'effectuer dans un réacteur continu ou discontinu. Lorsque la réaction met en oeuvre un système à deux phases différentes, les réacteurs continus peuvent être choisis parmi les réacteurs classiques tels que les réacteurs à lit fixe, les lits fluidisés ou les réacteurs à lit mobile permettant la circulation et la régénération éventuelle du catalyseur. Si trois phases sont présentes simultanément dans le système réactionnel, il est possible de faire usage des réacteurs continus conventionnels à lit fixe (trickle-bed) ou à slurry. Dans le cas d'un réacteur discontinu, on fait généralement usage d'un autoclave muni d'un agitateur à pales.

Le procédé selon l'invention est particulièrement bien adapté à la synthèse de tert-amylbenzène par alkylation de cumène avec de l'éthylène.

Les exemples qui suivent ont pour but d'illustrer l'invention sans en limiter sa portée.

### Exemples 1R et 2R (non conformes à l'invention)

Dans un autoclave d'une contenance de 400 ml muni d'un agitateur à pales, on a introduit 200 ml de cumène, 1 g d'hydrure de sodium, 0,5 g de KOH et une certaine quantité d'alumine.

L'autoclave a ensuite été fermé et chauffé à 170 °C pendant 15 minutes sous agitation et purge d'azote. On a ensuite démarré la réaction en injectant de l'éthylène sous une pression de 1 MPa dans le réacteur.

On a laissé la réaction se poursuivre pendant une heure, après quoi on a dosé le tert-amylbenzène formé et le cumène résiduel. Les résultats ont été portés au tableau qui suit.

| No de l'exemple | Rapport pondéral NaH/Al₂O₃ | Taux de conversion du cumène, % | Productivité g TAB/h.g cat. |
|---|---|---|---|
| 1R | 0,5 | 99,8 | 58,2 |
| 2R | 2,0 | 0,0 | 0,0 |

Dans ce tableau, le taux de conversion du cumène est égal à 100 fois le rapport molaire du cumène consommé au cumène mis en oeuvre, la productivité est égale au poids en g de tert-amylbenzène (TAB) formé par heure et par g de catalyseur.

### Exemples 3 à 5 (selon l'invention)

On a reproduit l'exemple 1 en faisant varier le poids d'alumine.

Les résultats obtenus sont donnés dans le tableau qui suit :

| No de l'exemple | Rapport Pondéral NaH/Al₂O₃ | Taux de conservation du cumène, % | Productivité g TAB/h.g cat. |
|---|---|---|---|
| 3 | 0,6 | 99,5 | 64,5 |
| 4 | 0,8 | 98,7 | 74,5 |
| 5 | 1,0 | 98,7 | 81,5 |

## Revendications

1. Procédé de synthèse d'hydrocarbures aromatiques alkylés à chaîne alkyle saturée comprenant au moins quatre atomes de carbone selon lequel on fait réagir un hydrocarbure aromatique substitué par un groupement alkyle à chaîne courte saturée comportant un à trois atomes de carbone avec une oléfine en présence d'un catalyseur qui comprend au moins un métal alcalin ou un hydrure de métal alcalin imprégné sur un support d'alumine, le catalyseur étant préparé dans le milieu réactionnel en présence de l'hydrocarbure aromatique à courte chaîne alkyle par mélange de l'alumine anhydre avec le métal alcalin ou avec l'hydrure de métal alcalin, caractérisé en ce que le rapport pondéral du métal alcalin ou de l'hydrure de métal alcalin au support d'alumine est compris entre 0,6 et 1,8.

2. Procédé selon la revendication 1, caractérisé en ce que le support d'alumine présente un diamètre moyen de pores compris entre 2 et 100 nm.

3. Procédé selon la revendication 1 ou 2, caractérisé en ce que le support d'alumine est constitué par de l'alumine γ.

4. Procédé selon l'une quelconque des revendications 1 à 3, caractérisé en ce que le support d'alumine présente un volume de pores compris entre 25 et 75 ml/100 g d'alumine et une surface spécifique comprise entre 10 et 360 m/g d'alumine.

5. Procédé selon l'une quelconque des revendications 1 à 4, caractérisé en ce que le rapport pondéral du métal alcalin ou de l'hydrure de métal alcalin à l'hydrocarbure aromatique à courte chaîne est situé dans la plage allant de 0,0015 à 0,03.

6. Procédé selon l'une quelconque des revendications 1 à 5, caractérisé en ce que le métal alcalin est du sodium et l'hydrure de métal alcalin de l'hydrure de sodium.

7. Procédé selon l'une quelconque des revendications 1 à 6, caractérisé en ce qu'on effectue la réaction à une température comprise entre 170 et 200 °C.

8. Utilisation du procédé selon l'une quelconque des revendications 1 à 7 pour la synthèse du tert-amylbenzène par alkylation de cumène avec de l'éthylène.

## Patentansprüche

1. Verfahren zur Synthese von aromatischen Kohlenwasserstoffen, die mit einer gesättigten Alkylkette, die wenigstens vier Kohlenstoffatome umfaßt, alkyliert sind, gemäß dem man einen aromatischen Kohlenwasserstoff, der mit einer Alkylgruppe mit kurzer gesättigter Kette, die ein bis drei Kohlenstoffatome umfaßt, substituiert ist, mit einem Olefin in Gegenwart eines Katalysators, der wenigstens ein Alkalimetall oder ein Alkalimetallhydrid aufgebracht auf einem Aluminiumoxidträger enthält, reagieren läßt, wobei der Katalysator im Reaktionsmedium in Gegenwart des aromatischen Kohlenwasserstoffs mit kurzer Alkylkette durch Mischen des wasserfreien Aluminiumoxids mit dem Alkalimetall oder mit dem Alkalimetallhydrid hergestellt wird, dadurch gekennzeichnet, daß das Gewichtsverhältnis des Alkalimetalls oder des Alkalimetallhydrids zu dem Aluminiumoxidträger zwischen 0,6 und 1,8 liegt.

2. Verfahren gemäß Anspruch 1, dadurch gekennzeichnet, daß der Aluminiumoxidträger einen mittleren Porendurchmesser zwischen 2 und 100 nm aufweist.

3. Verfahren gemäß Anspruch 1 oder 2, dadurch gekennzeichnet, daß der Aluminiumträger aus γ-Aluminiumoxid besteht.

4. Verfahren gemäß einem der Ansprüche 1 bis 3, dadurch gekennzeichnet, daß der Aluminiumoxidträger ein Porenvolumen zwischen 25 und 75 ml/100 g Aluminiumoxid und eine spezifische Oberfläche zwischen 10 und 360m/g Aluminiumoxid aufweist.

5. Verfahren gemäß einem der Ansprüche 1 bis 4, dadurch gekennzeichnet, daß das Gewichtsverhältnis des Alkalimetalls oder des Alkalimetallhydrids zu dem aromatischen Kohlenwasserstoff mit kurzer Kette im Bereich von 0,0015 bis 0,03 liegt.

6. Verfahren gemäß einem der Ansprüche 1 bis 5, dadurch gekennzeichnet, daß das Alkalimetall Natrium und das Alkalimetallhydrid Natriumhydrid ist.

7. Verfahren gemäß einem der Ansprüche 1 bis 6, dadurch gekennzeichnet, daß die Reaktion bei einer Temperatur zwischen 170 und 200 °C ausgeführt wird.

8. Verwendung des Verfahrens gemäß einem der Ansprüche 1 bis 7 zur Synthese von tert-Amylbenzol durch Alkylierung von Cumol mit Ethylen.

## Claims

1. Process for the synthesis of alkylated aromatic hydrocarbons containing a saturated alkyl chain comprising at least four carbon atoms, according to which an aromatic hydrocarbon substituted by a saturated short-chain alkyl group containing one to three carbon atoms is reacted with an olefin in the presence of a catalyst which comprises at least one alkali metal or one alkali metal hydride impregnated on an alumina support, the catalyst being prepared in the reaction medium in the presence of the aromatic hydrocarbon containing a short alkyl chain by mixing anhydrous alumina with the alkali metal or with the alkali metal hydride, characterized in that the ratio by weight of the alkali metal or of the alkali metal hydride to the alumina support is between 0.6 and 1.8.

2. Process according to Claim 1, characterized in that the alumina support has a mean pore diameter of between 2 and 100 nm.

3. Process according to Claim 1 or 2, characterized in that the alumina support consists of γ-alumina.

4. Process according to any one of Claims 1 to 3, characterized in that the alumina support has a pore volume of between 25 and 75 ml/100 g of alumina and a specific surface of between 10 and 360 m/g of alumina.

5. Process according to any one of Claims 1 to 4, characterized in that the ratio by weight of the alkali metal or of the alkali metal hydride to the short-chain aromatic hydrocarbon lies in the range ranging from 0.0015 to 0.03.

6. Process according to any one of Claims 1 to 5, characterized in that the alkali metal is sodium and the alkali metal hydride is sodium hydride.

7. Process according to any one of Claims 1 to 6, characterized in that the reaction is carried out at a temperature between 170 and 200°C.

8. Use of the process according to any one of Claims 1 to 7 for the synthesis of tert-amylbenzene by alkylation of cumene with ethylene.
